# FASCICULE DE BREVET EUROPEEN

(11) **EP 0 517 601 B1**
(45) Date de publication et mention de la délivrance du brevet: **21.09.1994**
(21) Numéro de dépôt: 92401545.6
(22) Date de dépôt: 04.06.1992
(51) Int. Cl.: G01T 1/166, A61B 6/00

(54) **Gamma caméra tomographique munie d'un détecteur orientable**
Tomographische Gammakamera mit ausrichtbarem Detektor
Tomographical gamma camera provided with a steerable detector

(30) Priorité: 07.06.1991 FR 9106960
(43) Date de publication de la demande: 09.12.1992
(73) Titulaire: SOPHA MEDICAL, F-75008 Paris (FR)
(72) Inventeur: Pierfitte, Michel, CABINET BALLOT-SCHMIT, F-75116 Paris (FR)
(74) Mandataire: Schmit, Christian Norbert Marie

(56) Documents cités:
- EP-A- 0 266 846
- US-A- 4 652 759
- PATENT ABSTRACTS OF JAPAN vol. 9, no. 289 (P-405)(2012) 15 Novembre 1985 & JP-A-60 128 383

## Description

La présente invention se rapporte aux dispositifs qui permettent d'orienter une tête détectrice d'une gamma caméra, de préférence tomographique, de manière convenable par rapport au patient qui subit un examen avec cette gamma caméra.

Des gamma caméras sont par exemple décrites dans le brevet américain de ANGER n° 3 011 057. Une gamma caméra est un appareil comportant une embase tournante, fixe ou mobile par rapport au sol, et qui porte, au bout d'un bras, un détecteur, dit encore tête détectrice. Ce détecteur est muni d'un réseau de tubes photo-multiplicateurs dont les faces d'entrée, juxtaposées les unes aux autres, constituent la surface de détection de la tête détectrice et son champ de détection.

Le principe de l'examen est le suivant. On injecte un produit radioactif dans un patient à examiner. Ce produit est par exemple du Thallium. L'émission radioactive vient exciter un cristal scintillateur du détecteur qui convertit l'énergie des photons gamma en une énergie lumineuse détectable par les tubes photo-multiplicateurs. Le cristal scintillateur est précédé, d'une manière classique, d'un collimateur définissant une direction de visée et caractérisé par un foyer. Ce foyer est rejeté à l'infini dans le cas des collimateurs à trous parallèles, droits ou inclinés. Le foyer est à une distance finie, positive ou négative, dans le cas des collimateurs convergents ou divergents. Le foyer peut être décentré par rapport à une direction centrale de visée.

Les scintillations émises sont détectées par les tubes photo-multiplicateurs qui produisent des signaux électriques dépendant de l'intensité lumineuse reçue. En effectuant sur l'ensemble de ces signaux électriques des localisations barycentriques, on peut, d'une manière connue, déterminer la localisation X Y de l'origine la scintillation dans le champ de détection. On réalise alors une acquisition incrémentale en cumulant le nombre de scintillations (ou coups) détectés par élément de localisation dit pixel.

En laissant la tête détectrice dans une position donnée pendant un certain temps au-dessus du corps examiné, on peut alors, pour un angle de vue donné, dit projection, obtenir une image révélatrice de la concentration du produit émetteur dans le corps. L'examen tomographique consiste à acquérir une image par angle de vue, pour un grand nombre d'angles de vue, régulièrement espacés sur un secteur angulaire d'au moins 180°. On sait ensuite, avec des algorithmes de calcul, notamment la rétro-projection filtrée, reconstituer l'image d'un volume du corps.

Le patient examiné est allongé sur un lit. Le lit est orientable par rapport à un bâti fixe, ou mobile en translation, qui supporte la gamma caméra par l'intermédiaire d'organes articulés. En combinant ces différents moyens, on peut ainsi orienter la gamma caméra par rapport au corps du patient pour obtenir l'image de l'organe souhaité. Pour des raisons claires de coût, il est nécessaire de limiter le plus possible le nombre d'articulations des organes qui soutiennent la gamma caméra, dans la limite des résultats à obtenir.

Jusqu'à une période récente, la partie sensible de la gamma caméra était ronde, ce qui rendait indifférent, toutes choses égales par ailleurs, l'orientation du patient autour de la normale à cette partie sensible. On, utilise maintenant des gamma caméras dont la tête détectrice n'a pas de symétrie de révolution. Sa forme peut être rectangulaire, à angles tronqués ou non, ou même elliptique. Elle est caractérisée par un grand axe et un petit axe, axes de symétrie dans le plan du détecteur. La dimension du grand axe est supérieure à celle du petit. Dans ces cas, l'orientation du patient autour de cette normale n'est plus indifférente, pour utiliser au mieux le champ de détection. Cette exigence complique encore les articulations à prévoir.

Pour orienter au mieux le champ de détection de la gamma caméra, le document EP-A-0 266 846 propose un mouvement d'orientation autour d'un axe perpendiculaire au champ de détection de la tête détectrice. Le mouvement est réalisé par une rotation succesive ou simultanée autour de deux axes situés dans le champ de détection et ne permet qu'une orientation d'exactement 90°.. Il est donc possible d'aligner le petit axe de la tête suivant l'axe du patient, que celui-ci soit présenté dans l'axe de rotation de l'embase de la gamma caméra ou perpendiculairement à ce dernier.

L'invention a pour objet une gamma caméra du type comportant un bâti, une embase tournante reliée à ce bâti par un axe de rotation permettant une rotation de l'embase tournante autour de cet axe, un bras relié à l'embase tournante par des moyens permettant un mouvement radial du bras par rapport à cet axe de rotation, une tête détectrice de gamma caméra maintenue par ce bras, caractérisée en ce qu'elle comprend en outre, dans le maintien de la tête détectrice, un support intermédiaire fixé au bras par un axe d'orientation, perpendiculaire à l'axe de rotation, cet axe d'orientation permettant d'orienter cette tête détectrice par rapport au bras, et cette tête détectrice étant fixée au support intermédiaire par un axe d'angulation perpendiculaire à l'axe d'orientation et permettant, lorsque cet axe d'angulation est orienté parallèlement à l'axe de rotation, une angulation (Y) de cette tête détectrice de telle façon qu'une direction principale de visée de cette tête soit contenue dans un plan perpendiculaire à l'axe de rotation.

Quand la gamma caméra comporte un axe d'angulation permettant de donner une angulation à la tête détectrice, l'axe d'orientation est perpendiculaire à l'axe de rotation et à l'axe d'angulation, et il relie un étrier à l'extrémité du bras, cet étrier permettant d'orienter la tête par rapport au bras .

D'autres particularités et avantages de l'invention apparaîtront clairement dans la description suivante, faite à titre d'exemple non limitatif en regard des figures annexées qui représentent :
- La figure 1, un dispositif d'angulation représentant en partie l'art connu ; et
- la figure 2, un dispositif selon l'invention, limité aux moyens nouveaux de l'invention.

La présente invention est déposée comme demande de brevet en même temps que deux autres inventions du même inventeur qui portent sur d'autres perfectionnements.

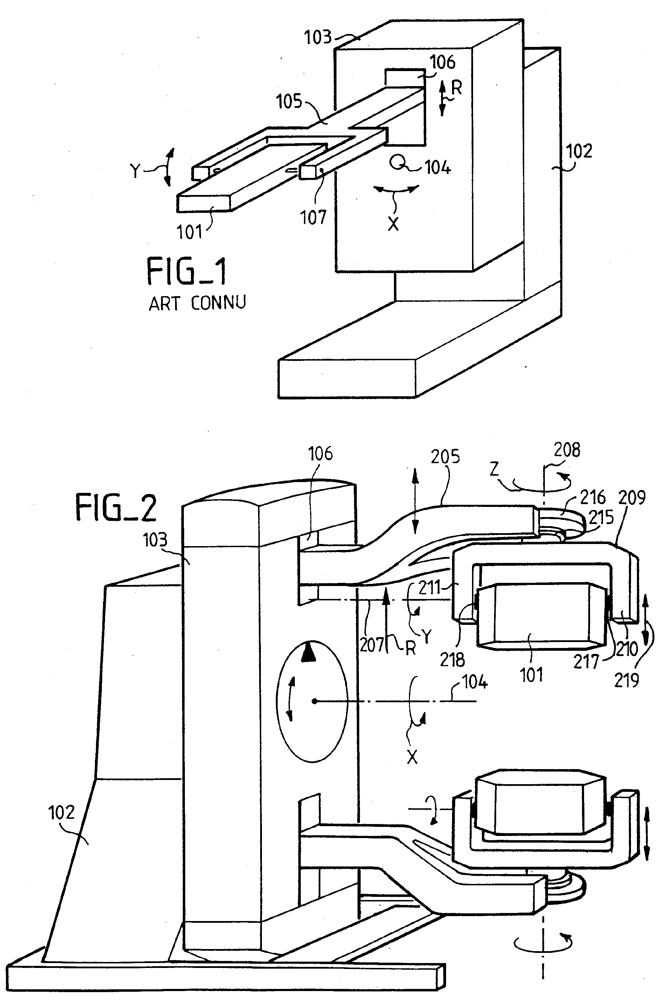

Dans le dispositif selon l'art connu représenté en figure 1, une tête détectrice 101 d'une gamma caméra est maintenue de manière à subir une angulation sur un bâti 102 en forme de L, qui repose sur le sol. Une embase tournante 103 est fixée au bras vertical du L par un axe horizontal 104 qui permet à l'embase tournante d'effectuer un mouvement de rotation X autour de cet axe. Conventionnellement ce mouvement est appelé rotation de la gamma caméra.

Un bras 105 en forme de fourchette est fixé du côté manche à l'embase tournante 103 à l'intérieur d'une fenêtre 106. Un mécanisme connu non représenté permet de faire subir à ce bras un mouvement de translation radiale R le long de l'embase tournante à l'intérieur de la fenêtre, de manière à l'éloigner ou à le rapprocher de l'axe 104.

La tête détectrice 101 de la gamma caméra, qui est rectangulaire, est fixée entre les deux "doigts" de la fourchette sur un axe 107, lequel est orthogonal à l'axe 104, et est horizontal lorsque l'embase tournante est verticale (la translation R est alors verticale). Cet axe permet de faire subir à la tête détectrice un mouvement de rotation Y, qui est conventionnellement appelé angulation.

Le patient repose sur un lit qui est placé sous la tête détectrice, parallèlement ou perpendiculairement à l'axe 104 selon les besoins de l'examen. La gamma caméra est elle-même orientée par translation R, rotation X et angulation Y selon ces besoins.

Les différentes combinaisons de ces diverses dispositions et mouvements sont limitées. On se trouve donc parfois dans une situation où la grande dimension de la gamma caméra déborde du corps du patient en laissant une zone inutilisée, ou au contraire où la plus petite dimension de cette gamma caméra est plus petite que la partie du corps à examiner. Entre autres inconvénients, cette situation prolonge la durée de l'examen, ce qui est pénible pour le patient et fait baisser la productivité de l'appareillage.

Le dispositif selon la présente invention, qui permet de résoudre ce problème, est représenté sur la figure 2 d'une manière limitée aux moyens spécifiques et nouveaux de l'invention. Plus particulièrement, un bras 205 est fixé d'un côté sur l'embase tournante de manière mobile pour obtenir le mouvement de translation R, comme sur la figure 1. Ce bras porte à son autre extrémité un axe 208 qui est parallèle à la translation radiale R, et est vertical lorsque l'embase tournante est verticale (la translation R est alors verticale). Cette translation radiale s'effectue suivant une direction principale de l'embase 103. Cet axe 208 est perpendiculaire à l'axe 104. Il porte un étrier 209 en forme de U dont les deux flancs latéraux 210 et 211 lui sont parallèles (verticaux quand l'embase tournante est verticale). Cet axe 208 permet de faire subir à l'étrier, et donc à la tête détectrice qui lui est fixée, un mouvement de rotation Z, appelé conventionnellement orientation.

La tête détectrice 101 de la gamma caméra est fixée à l'intérieur de l'étrier 209, entre les deux flancs du U, par un axe 207, perpendiculaire à l'axe 208. Cet axe 207 est donc horizontal lorsque l'embase tournante est verticale et il permet de faire subir à la tête le mouvement d'angulation Y défini plus haut par rapport à la figure 1. Afin de permettre un débattement suffisant sans allonger excessivement les bras latéraux de l'étrier la gamma caméra est fixée de préférence de telle manière que sa plus grande dimension, son grand axe, soit parallèle à l'axe 207.

Dans ces conditions, on remarque que cette grande dimension de la tête de la gamma caméra, telle qu'elle est représentée sur la figure 2, est présentée comme celle de la figure 1. Pour obtenir une disposition à 90°, il suffit de faire tourner l'étrier autour de l'axe 208 d'un angle de 90 degrés, c'est-à-dire conventionnellement de lui faire subir une orientation de 90 degrés.

Ainsi donc l'axe 208 permet de donner à la gamma caméra un degré supplémentaire de liberté en orientation pour obtenir un meilleur positionnement relatif entre celle-ci et le patient qui subit l'examen.

La description ci-dessus a été faite dans le cas où il n'y a qu'une seule tête détectrice fixée sur l'embase tournante par les moyens décrits. Elle s'étend bien entendu au cas où l'on utilise deux têtes détectrices fixées aux deux extrémités de l'embase tournante et donc placées de part et d'autre du patient (l'une au dessus et l'autre au dessous lorsque l'embase tournante est verticale) comme cela est suggéré sur la figure 2.

Les deux têtes sont maintenues à l'extrémité de leur bras de la même façon. Par exemple, la tête 101 est munie de deux axes 217 et 218 qui peuvent tourner dans des paliers en maintenant la tête. Ces paliers eux mêmes sont maintenus dans des flancs respectivement 210 et 211 de l'étrier 209. Le sommet de l'étrier 209 est relié à une couronne 215 qui peut tourner à l'intérieur d'une couronne concentrique 216 qui elle même est fixée à l'extrémité du bras 205.

L'étrier 209, qui a une forme de U renversé, comprend, dans chacun des ses flancs 210 et 211, un chariot coulissant pour permettre aux paliers qui maintiennent les axes 217 et 218 de se mouvoir verticalement. Ce mouvement de coulissement est de préférence motorisé par un moteur placé dans chaque étrier. Les moteurs sont indépendants. Un moteur est par exemple placé au centre de l'étrier 209. Il entraîne, par deux courroies passant dans le sommet de l'étrier, deux vis sans fin parallèles aux flans 210 et 211. Une vis sans fin se visse dans un écrou solidaire d'un chariot. Les chariots sont munis de deux douilles qui coulissent le long de deux arbres. Les arbres sont maintenus solidaires des flans de l'étrier. La tête de la vis sans fin est maintenue fixe en translation mais libre en rotation dans l'étrier. Le mouvement télescopique est représenté symboliquement par la flèche double 219 sur la figure 2.

Dans une version simplifiée le mouvement d'angulation est manuel avec possibilité de sélection parmi des positions prédéfinies. Dans un exemple ces position prédéfinies sont constituées par des encoches réalisées en périphérie de plaques circulaires concentriques à l'axe d'angulation et solidaires de chacune des têtes. Deux crans peuvent venir s'engager dans ces encoches et ainsi maintenir dans des positions prédéfinies l'angulation des têtes.

Les positions en orientation de l'étrier peuvent être quelconques, ainsi le sommet de l'étrier 209 est relié à une couronne 215 qui peut tourner à l'intérieur d'une couronne concentrique 216 qui elle même est fixée à l'extrémité du bras 205. Les profils des couronnes 15 et 16 sont entrelacés de manière à provoquer le maintien de la tête dans toutes les positions. Lorsque la couronne 215 tourne à l'intérieur de la couronne 216 la tête tourne d'un mouvement d'orientation autour de l'axe 208. Certaines positions en orientation sont aussi de préférence prédéfinies: par exemple les profils des couronnes 215 et 216 peuvent comporter des bossages et respectivement des cavités régulièrement réparties sur leur circonférence qui, lorsqu'elles se rencontrent s'imbriquent l'un dans l'autre par réaction élastique. Ces imbrications sont suffisantes pour assurer le maintien en orientation, elles peuvent en étant forcées conduire à une autre orientation. L'orientation peut aussi être motorisée et dans ce cas le moteur lui même assure le maintien en orientation.

## Revendications

1. Gamma caméra du type comportant un bâti (102), une embase tournante (103) reliée à ce bâti par un axe (104) de rotation permettant une rotation (X) de l'embase tournante autour de cet axe, un bras (205) relié à l'embase tournante par des moyens (106) permettant un mouvement radial (R) du bras par rapport à cet axe de rotation, une tête détectrice de gamma caméra (101) maintenue par ce bras, la caméra comprenant en outre, dans le maintien de la tête détectrice, un support intermédiaire fixé au bras par un axe (208) d'orientation, perpendiculaire à l'axe de rotation, cet axe d'orientation permettant d'orienter (Z) cette tête détectrice par rapport au bras, et cette tête détectrice étant fixée au support intermédiaire par un axe (207) d'angulation perpendiculaire à l'axe d'orientation et permettant, lorsque cet axe d'angulation est orienté parallèlement à l'axe de rotation, une angulation (Y) de cette tête détectrice de telle façon qu'une direction principale de visée de cette tête soit contenue dans un plan perpendiculaire à l'axe de rotation.

2. Gamma caméra selon la revendication 1, caractérisée en ce que la tête détectrice est maintenue au bras par un étrier (209) auquel est reliée la tête détectrice par l'axe d'angulation, l'axe (208) d'orientation étant perpendiculaire à l'axe de rotation et à l'axe d'angulation.

3. Dispositif selon la revendication 1 ou la revendication 2, caractérisé en ce que la tête détectrice est fixée au bras par un étrier (209) en forme de U à l'intérieur duquel elle est placée, et en ce que l'axe d'orientation (208) est fixé sur l'étrier sensiblement au centre du bras transversal de ce U.

4. Dispositif selon l'une quelconque des revendications 1 à 3, caractérisé en ce que la tête détectrice (101) est de forme rectangulaire et que sa plus grande dimension est parallèle à l'axe d'angulation.

5. Dispositif selon l'une quelconque des revendications 1 à 4, caractérisé en ce qu'il comprend en outre un deuxième ensemble formé d'un bras (205), d'un étrier (209), d'une tête détectrice (201) et de deux axes (207, 208), ce deuxième ensemble étant fixé sur l'embase tournante de l'autre côté de l'axe de rotation (104) symétriquement par rapport a des organes homologues formant un premier ensemble.

## Patentansprüche

1. Gammakamera, umfassend ein Gestell (102), einen rotierenden Aufsatz (103), der mit diesem Gestell durch eine Rotationsachse (104) verbunden ist, die eine Rotation (X) des rotierenden Aufsatzes um diese Achse gestattet, einen Arm (205), der mit dem rotierenden Aufsatz durch Mittel (106) verbunden ist, die eine radiale Bewegung (R) des Arms bezüglich dieser Rotationsachse gestatten, und einen von diesem Arm gehaltenen Gammakamera-Erfassungskopf (101), wobei die Kamer außerdem im Halt des Erfassungskopfs einen Zwischenhalter umfaßt, der am Arm durch eine zur Rotationsachse senkrechte Schwenkachse (208) befestigt ist, die die Verschwenkung (Z) dieses Erfassungskopfes bezüglich des Arms gestattet, wobei dieser Erfassungskopf an dem Zwischenhalter durch eine zur Schwenkachse senkrechte Angulierungsachse (207) befestigt ist, die, wenn diese Angulierungsachse parallel zur Rotationsachse gerichtet ist, eine Angulierung (Y) dieses Erfassungskopfes gestattet, so daß eine Hauptvisierrichtung dieses Kopfes in einer zur Rotationsachse senkrechten Ebene enthalten ist.

2. Gammakamera nach Anspruch 1, dadurch gekennzeichnet, daß der Erfassungskopf an dem Arm durch einen Bügel (209) gehalten ist, mit dem der Erfassungskopf durch die Angulierungsachse verbunden ist, wobei die Schwenkachse (208) zur Rotationsachse und zur Angulierungsachse senkrecht ist.

3. Vorrichtung nach Anspruch 1 oder Anspruch 2, dadurch gekennzeichnet, daß der Erfassungskopf an dem Arm durch einen U-förmigen Bügel (209) befestigt ist, in dessen Inneren er angeordnet ist, und daß die Schwenkachse (208) an dem Bügel im wesentlichen in der Mitte des Querarms dieses U befestigt ist.

4. Vorrichtung nach einem der Ansprüche 1 bis 3, dadurch gekennzeichnet, daß der Erfassungskopf (101) eine rechtekkige Form hat und daß seine größte Abmessung zur Angulierungsachse parallel ist.

5. Vorrichtung nach einem der Ansprüche 1 bis 4, dadurch gekennzeichnet, daß sie außerdem eine zweite aus einem Arm (205), einem Bügel (209), einem Erfassungskopf (201) und zwei Achsen (207, 208) bestehende Einheit umfaßt, die an dem rotierenden Aufsatz bezüglich entsprechender Organen, die eine erste Einheit bilden, symmetrisch auf der anderen Seite der Rotationsachse (104) befestigt ist.

## Claims

1. Gamma camera of the type comprising a frame (102), a rotary base (103) connected to the said frame by a rotation axis (104) enabling the rotary base to perform a rotation (X) about the said axis, an arm (205) connected to the rotary base by means (106) enabling the arm to perform radial movement (R) in relation to the said rotation axis, a gamma camera detector head (101) held by the said arm, the camera also comprising, in holding the detector head, an intermediate support affixed to the arm by an orientation axis (208) perpendicular to the rotation axis, this orientation axis enabling the said detector head to be adjusted (Z) in relation to the arm, and the said detector head being affixed to the intermediate support by an angulation axis (207) perpendicular to the orientation parallel to the rotation axis, to effect an angulation (Y) of the said detector head in such a manner that a main viewing direction of the said head will be contained within a plane perpendicular to the rotation axis.

2. Gamma camera in accordance with claim 1, characterised by the fact that the detector head is secured on the arm by a strap (209) to which the detector head is connected by the angulation axis, the orientation axis (208) being perpendicular to the rotation axis and to the angulation axis.

3. Device in accordance with claim 1 or claim 2, characterised by the fact that the detector head is affixed to the arm by a strap (209) of the shape of a U and is positioned inside the latter and that the orientation axis (208) is affixed to the strap in a position approximately in the centre of the transversal arm of the said U.

4. Device in accordance with any one of claims 1 to 3 characterised by the fact that the detector head (101) is rectangular in shape and that its long sides are parallel to the angulation axis.

5. Device in accordance with any one of claims 1 to 4 characterised by the fact that it also comprises a second assembly formed by an arm (205), a strap (209), a detector head (201) and two axes (207, 208), this second assembly being affixed to the rotary base on the other side of the rotation axis (14) and symmetrically in relation to analogous components forming a first assembly.
